(19) Europäisches Patentamt
European Patent Office
Office européen des brevets

(11) Veröffentlichungsnummer: 0 303 569
A2

# (12) EUROPÄISCHE PATENTANMELDUNG

(21) Anmeldenummer: 88810531.9

(22) Anmeldetag: 03.08.88

(51) Int. Cl.⁴: **C 07 D 327/06**
C 07 D 327/02, C 10 M 135/00

(30) Priorität: 12.08.87 CH 3097/87

(43) Veröffentlichungstag der Anmeldung: 15.02.89 Patentblatt 89/07

(84) Benannte Vertragsstaaten: DE FR GB IT

(71) Anmelder: CIBA-GEIGY AG
Klybeckstrasse 141
CH-4002 Basel (CH)

(72) Erfinder: Kristen, Ulrich, Dr.
Störklingasse 40
CH-4125 Riehen (CH)

Wirth, Hermann O., Dr.
Lessingstrasse 24
D-6140 Bensheim 3 (DE)

(54) **1,4-Oxathianone und 1,4-Oxathiepanone und deren Verwendung als Additive für funktionelle Flüssigkeiten.**

(57) Die Verbindungen der Formel

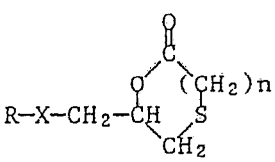

worin R $C_1$-$C_{24}$-Alkyl, $C_3$-$C_{20}$-Alkenyl, $C_5$-$C_6$-Cycloalkyl, unsubstituiertes oder durch $C_1$-$C_4$-Alkyl substituiertes Phenyl oder Phenyl-$C_1$-$C_4$-alkyl, n 1 oder 2 und X S oder O bedeuten, eignen sich hervorragend als Additive, insbesondere als Verschleissschutz- und Hochdruckadditive und als Korrosionsinhibitoren in funktionellen Flüssigkeiten, z.B. in Schmiermitteln, Kraftstoffen, Hydraulikflüssigkeiten, Metallbearbeitungsflüssigkeiten und Bohrflüssigkeiten.

EP 0 303 569 A2

**Beschreibung**

**1,4-Oxathianone und 1,4-Oxathiepanone und deren Verwendung als Additive für funktionelle Flüssigkeiten**

Die vorliegende Erfindung betrifft substituierte 1,4-Oxathianone und 1,4-Oxathiepanone, deren Verwendung als Additive für funktionelle Flüssigkeiten und Zusammensetzungen, die solche Flüssigkeiten und mindestens eine der genannten Verbindungen enthalten.

Schmierstoffen und Hydraulikflüssigkeiten werden im allgemeinen verschiedene Zusatzstoffe zur Verbesserung ihrer Gebrauchseigenschaften beigegeben. Da Schmierstoffe zur Uebertragung grösserer Kräfte ein hohes Lasttragevermögen benötigen, werden diesen sogenannte Hochdruck- und Antiverschleiss-Additive zugesetzt, wodurch die sonst auftretenden Verschleisserscheinungen stark erniedrigt werden. Wenn andererseits z.B. Sauerstoff und Feuchtigkeit gleichzeitig auf eine Metalloberfläche einwirken, kann Korrosion auftreten, weshalb Korrosionsinhibitoren mit dem Ziel zugegeben werden, den Zutritt solcher Stoffe zur Metalloberfläche zu verhindern. Die beispielsweise bei erhöhter Temperatur verstärkt durch Luftsauerstoff eintretenden Oxidationsreaktionen in einem Schmierstoff können durch Zugabe von Antioxidantien unterbunden werden. Es ist bekannt, dass bestimmte Stoffe als Additive für Schmierstoffe eine Anzahl derartiger Eigenschaften in sich vereinigen können; sie werden als sogenannte Vielzweck-Additive bezeichnet. Solche Stoffe sind natürlich aus ökonomischen und praktischen Gründen sehr gefragt.

In der Praxis werden heute verschiedene Phosphorverbindungen als Hochdruck- und Verschleisschutzadditive eingesetzt, insbesondere Zinkdialkyldithiophosphate (ZDTP). Besonders in Motorenölen von Benzinmotoren treten durch die Verwendung von P-haltigen Additiven Probleme auf, da sie die Wirksamkeit eines Abgasreinigungskatalysators herabsetzen können. Daher besteht ein Bedarf an phosphorarmen bzw. phosphorfreien Oeladditiven.

Derartige Additive wurden bereits in grosser Zahl vorgeschlagen. Die EP-A 166 696 beispielsweise beschreibt Umsetzungsprodukte von Glycidylthioethern mit Mercaptoverbindungen, die als Hochdruck- und Verschleisschutzadditve eingesetzt werden können.

Es wurden nun Verbindungen gefunden, die nicht nur sehr gute Hochdruck-und Verschleisschutzwirkung zeigen, sondern die auch universell einsetzbar sind. Sie zeigen zudem gute Korrosionsschutzwirkung. Ausserdem ist ihr Einsatz nicht auf hydrophobe funktionelle Flüssigkeiten beschränkt. Sie können ebenso in wässrigen oder organisch/wässrigen Systemen eingesetzt werden.

Die erfindungsgemässen Verbindungen entsprechen der Formel

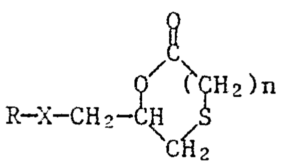

(I)

worin R $C_1$-$C_{24}$-Alkyl, $C_3$-$C_{20}$-Alkenyl, $C_5$-$C_6$-Cycloalkyl, unsubstituiertes oder durch $C_1$-$C_4$-Alkyl substituiertes Phenyl oder Phenyl-$C_1$-$C_4$-alkyl, n 1 oder 2 und X S oder O bedeuten.

Die Alkylgruppen R haben vorzugsweise 2 - 18, z.B. 2 - 16, insbesondere 4 - 18, z.B. 4 - 12 C-Atome. Besonders als Alkylreste sind tertiäre Alkylreste, beispielsweise tert-Butyl, tert.-Nonyl und tert-Dodecyl bevorzugt. Substituierte Phenylreste können 1 - 3 Alkylgruppen tragen, vorzugsweise 1 oder 2, vor allem Methylgruppen. Phenylalkyl ist insbesondere Phenethyl, α-Methylbenzyl, α,α-Dimethylbenzyl und vor allem Benzyl. Cycloalkyl ist bevorzugt Cyclohexyl.

In den Verbindungen der Formel I ist n vorzugsweise die Zahl 1.

Besonders zu erwähnen sind Verbindungen der Formel I, worin R $C_1$-$C_{18}$-Alkyl, $C_3$-$C_{18}$-Alkenyl, Phenyl oder Benzyl bedeutet, vor allem solche, worin R $C_4$-$C_{18}$-Alkyl, Phenyl oder Benzyl ist.

Von besonderem praktischem Interesse sind jene Verbindungen der Formel I, worin R $C_4$-$C_{18}$-Alkyl bedeutet, wobei solche Alkylgruppen bevorzugt sind, die mindestens ein tertiäres C-Atom enthalten.

Besonders bevorzugt sind jene Verbindungen der Formel (I), worin X S bedeutet.

Die erfindungsgemässen Verbindungen der Formel I könen nach an sich bekannten Verfahren hergestellt werden.

Eine Möglichkeit besteht in der intramolekularen Veresterung (Lactonisierung) von Verbindungen der Formel

$$R-X-\underset{OH}{CH}-CH_2-S-(CH_2)_n-COOMe \qquad II$$

worin Me das Aequivalent eines Alkali- oder Erdalkalimetallions, insbessondere aber Na, K oder Li, vor allem Na, bedeutet, mit Hilfe von sauren Katalysatoren. Als letztere können organische oder anorganische Säuren eingesetzt werden, beispielsweise eine Halogenwasserstoffsäure, Sauerstoffsäure des Schwefel oder

Phosphors, z.B. Schwefelsäure, Sulfonsäuren (z.B. Benzolsulfonsäure, p-Toluolsulfonsäure), Ameisensäure, Essigsäure usw. Es können aber auch saure Ionenaustauscher verwendet werden. Insbesondere ist es vorteilhaft, zur Vervollständigung der Reaktion einen sauren Ionenaustauscher zu verwenden, wenn auch vorher mit einer anderen Säure angesäuert wurde.

Die Reaktion kann durch das Schema

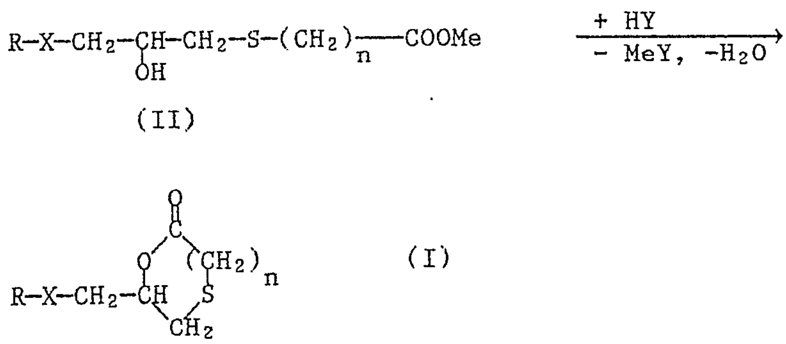


(II)

(I)

verdeutlicht werden. Y bedeutet z.B. Cl oder 1/2 $H_2SO_4$. Die Entfernung des gebildeten Wassers kann nach üblichen Methoden erfolgen, z.B. mit Hilfe eines Rotationsverdampfers.

Grundsätzlich ist es auch möglich, die Wasserabscheidung mit einem geeigneten Lösungsmittel, wie etwa Toluol, in einer entsprechenden Apparatur (azeotrope Destillation mit Wasserabscheidern) vorzunehmen. Auch hier wird die Reaktion in Gegenwart eines sauren Katalysators, z.B. der p-Toluolsulfonsäure oder besonders zweckmässig eines sauren Ionenaustauschers, durchgeführt.

Eine weitere Möglichkeit besteht in der intramolekularen Veresterung eines der Formel II entsprechenden Esterderivates unter Abspaltung des Alkohols nach dem Schema

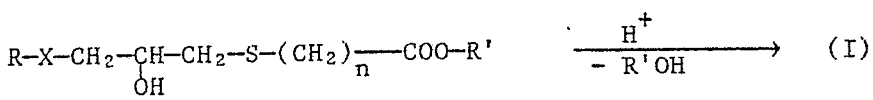


(I)

(III)

wobei R′ Alkyl ist.

Als Säurekatalysatoren kommen ebenfalls die vorstehend angeführten in Betracht. Die Entfernung des Alkohols kann analog zur Entfernung des Wassers in der erstgenannten Methode erfolgen. Beide Verfahren kann man mit oder ohne Lösungsmittel durchführen. Vorzugsweise wird die intramolekulare Veresterung direkt in der Reaktionsmischung ausgeführt, in der die Ausgangsstoffe der Formeln II und III hergestellt werden.

Diese Ausgangsprodukte können z.B. nach dem Schema

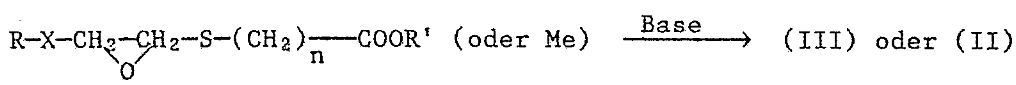


(III) oder (II) erhalten werden. Diese Reaktion wird in Gegenwart einer Base, insbesondere bei pH-Werten um 13 und vorzugsweise ohne zusätzliche Lösungsmittel oder in wässerigem Medium durchgeführt. Einzelheiten über die Reaktion als solche und bevorzugte Reaktionsparameter können der EP-A 166 696 entnommen werden. Besonders vorteilhafte Basen sind tertiäre Amine wie Tri($C_1$-$C_4$-alkyl)amine und cyclische tertiäre Amine wie Pyridin.

Eine weitere Möglichkeit zur Herstellung der Verbindungen der Formeln II und III basiert auf folgender Reaktion:

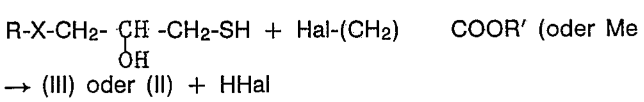

→ (III) oder (II) + HHal

Hal bedeutet Halogen, insbesondere Cl. Vorteilhaft wird für diese Umsetzung die Phasentransfertechnik

angewendet. Als HHal-Acceptor wird zweckmässig eine Base, z.B. NaOH eingesetzt. Die Reaktion lässt sich aber auch in rein organischem Medium durchführen (Lösungsmittel z.B. Toluol, Dibutylether usw.), wobei als Halogenwasserstoffacceptor zweckmässig tertiäre Amine verwendet werden.

Die erfindungsgemässen Verbindungen der Formel I sind von flüssiger bis viskoser Konsistenz. Sie sind in unpolaren organischen Substanzen (z.B. in Schmierölen) sehr gut löslich. Sie können jedoch auch in polare Systeme (z.B. auf Basis von Wasser und/oder Alkoholen) eingebracht werden und dort ihre Wirkung entfalten, wobei zumindest teilweise eine Oeffnung des Lactonringes erfolgt. Zu diesem Zweck kann es vorteilhaft sein, solchen wässrigen Systemen kleine Mengen von Basen zuzusetzen.

Auf Grund dieser Eigenschaften sind die erfindungsgemässen Verbindungen ausserordentlich vielfältig anwendbar in allen Arten von funktionellen Flüssigkeiten, ob sie nun auf unpolaren (z.B. öligen) Flüssigkeiten oder auf (teilweise) polaren (z.B. wässrigen) Systemen beruhen. Als funktionelle Flüssigkeiten, in denen die erfindungsgemässen Verbindungen eingesetzt werden können, seien beispielsweise erwähnt: Schmierstoffe, Kraftstoffe, Hydraulikflüssigkeiten, Metallbearbeitungsflüssigkeiten, Kühlflüssigkeiten und Bohrflüssigkeiten. Die Verbindungen zeigen sehr gute Stabilisatoreigenschaften, insbesondere Verschleisschutz- und Hochdruck-Eigenschaften, haben aber zusätzlich noch den Vorteil einer ausgeprägten Korrosionsschutzwirkung.

Die Verbindungen der Formel I wirken schon in sehr geringen Mengen als Additive in Schmierstoffen. Sie werden den Schmierstoffen zweckmässigerweise in einer Menge von 0,01 bis 5 Gew.-%, vorzugsweise in einer Menge von 0,05 bis 3 Gew.-%, bezogen auf den Schmierstoff, zugesetzt. Die in Frage kommenden Schmierstoffe sind dem Fachmann geläufig und z.B. in "Schmierstoffe und verwandte Produkte" (Verlag Chemie, Weinheim, 1982) beschrieben. Besonders geeignet sind neben Mineralölen z.B. Poly-α-Olefine, Schmierstoffe auf Esterbasis, Phosphate, Glykole, Polyglykole und Polyalkylenglykole.

Die Schmierstoffe können zusätzlich andere Additive enthalten, die zugegeben werden, um die Grundeigenschaften von Schmierstoffen noch weiter zu verbessern; dazu gehören: Antioxidantien, Metallpassivatoren, Rostinhibitoren, Viskositätsindex-Verbesserer, Stockpunkterniedriger, Dispergiermittel, Detergentien, weitere Hochdruck-Zusätze und Antiverschleiss-Additive. Im folgenden sind einige Beispiele von derartigen zusätzlichen Additiven aufgezählt:

<u>Beispiele für phenolische Antioxidantien</u>

<u>1. Alkylierte Monophenole</u>
2,6-Di-tert-butyl-4-methylphenol
2,6-Di-tert-butylphenol
2-tert-Butyl-4,6-dimethylphenol
2,6-Di-tert-butyl-4-ethylphenol
2,6-Di-tert-butyl-4-n-butylphenol
2,6-Di-tert-butyl-4-iso-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert-butyl-4-methoxymethylphenol
= o-tert-Butylphenol

<u>2. Alkylierte Hydrochinone</u>
2,6-Di-tert-butyl-4-methoxyphenol
2,5-Di-tert-butyl-hydrochinon
2,5-Di-tert-amyl-hydrochinon
2,6-Diphenyl-4-octadecyloxyphenol

<u>3. Hydroxylierte Thiodiphenylether</u>
2,2′-Thio-bis-(6-tert-butyl-4-methylphenol)
2,2′-Thio-bis-(4-octylphenol)
4,4′-Thio-bis-(6-tert-butyl-3-methylphenol)
4,4′-Thio-bis-(6-tert-butyl-2-methylphenol)

<u>4. Alkyliden-Bisphenole</u>
2,2′-Methylen-bis-(6-tert-butyl-4-methylphenol)
2,2′-Methylen-bis-(6-tert-butyl-4-ethylphenol)
2,2′-Methylen-bis-[4-methyl-6-(α-methylcyclohexyl)-phenol]
2,2′-Methylen-bis-(4-methyl-6-cyclohexylphenol)

2,2′-Methylen-bis-(6-nonyl-4-methylphenol)
2,2′-Methylen-bis-(4,6-di-tert-butylphenol)
2,2′-Ethyliden-bis-(4,6-di-tert-butylphenol)
2,2′-Ethyliden-bis-(6-tert-butyl-4-iso-butylphenol)
2,2′-Methylen-bis-[6-(α-methylbenzyl)-4-nonylphenol]
2,2′-Methylen-bis-[6-(α,α-dimethylbenzyl)-4-nonylphenol]
4,4′-Methylen-bis-(2,6-di-tert-butylphenol)
4,4′-Methylen-bis (6-tert-butyl-2-methylphenol)
1,1-Bis-(5-tert-butyl-4-hydroxy-2-methylphenyl)-butan
2,6-Di-(3-tert-butyl-5-methyl-2-hydroxybenzyl)-4-methylphenol
1,1,3-Tris-(5-tert-butyl-4-hydroxy-2-methylphenyl)-3-n-dodecylmercaptobutan
Ethylenglycol-bis-[3,3-bis-(3′-tert-butyl-4′-hydroxyphenyl)-butyrat]
Di-(3-tert-butyl-4-hydroxy-5-methylphenyl)-dicyclopentadien
Di-[2-(3′-tert-butyl-2′-hydroxy-5′-methyl-benzyl)-6-tert-butyl-4-methylphenyl]-terephthalat.

5. Benzylverbindungen
1,3,5-Tri-(3,5-di-tert-butyl-4-hydroxybenzyl)-2,4,6-trimethylbenzol
Di-(3,5-di-tert-butyl-4-hydroxybenzyl)-sulfid
3,5-Di-tert-butyl-4-hydroxybenzyl-mercaptoessigsäure-isooctylester
Bis-(4-tert-butyl-3-hydroxy-2,6-dimethylbenzyl)-dithiol-terephthalat
1,3,5-Tris-(3,5-di-tert-butyl-4-hydroxybenzyl)-isocyanurat
1,3,5-Tris-(4-tert-butyl-3-hydroxy-2,6-dimethylbenzyl)-isocyanurat
3,5-Di-tert-butyl-4-hydroxybenzyl-phosphonsäure-dioctadecylester
3,5-Di-tert-butyl-4-hydroxybenzyl-phosphonsäure-monoethylester
Calcium-salz.

6. Acylaminophenole
4-Hydroxy-laurinsäureanilid
4-Hydroxy-stearinsäureanilid
2,4-Bis-octylmercapto-6-(3,5-di-tert-butyl-4-hydroxyanilino)-s-triazin
N-(3,5-di-tert-butyl-4-hydroxyphenyl)-carbaminsäureoctylester.

7. Ester der β-(3,5-Di-tert-butyl-4-hydroxyphenyl)-propionsäure
mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyl-oxalsäurediamid

8. Ester derβ-(5-tert-butyl-4-hydroxy-3-methylphenyl)-propion säure
mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyl-oxalsäurediamid

9. Amide der β-(3,5-Di-tert-butyl-4-hydroxyphenyl)-propionsäure,
wie z.B.
N,N′-Di-(3,5-di-tert-butyl-4-hydroxyphenylpropionyl)-hexamethylendiamin
N,N′-Di-(3,5-di-tert-butyl-4-hydroxyphenylpropionyl)-trimethylendiamin
N,N′-Di-(3,5-di-tert-butyl-4-hydroxyphenylpropionyl)-hydrazin.

Beispiele für aminische Antioxidantien:
N,N′-Di-isopropyl-p-phenylendiamin
N,N′-Di-sec-butyl-p-phenylendiamin
N,N′-Bis(1,4-dimethyl-pentyl)-p-phenylendiamin
N,N′-Bis(1-ethyl-3-methyl-pentyl)-p-phenylendiamin

N,N′-Bis(1-methyl-heptyl)-p-phenylendiamin
N,N′-Diphenyl-p-phenylendiamin
N,N′-Di-(naphthyl-2)-p-phenylendiamin
N-Isopropyl-N′-phenyl-p-phenylendiamin
N-(1,3-Dimethyl-butyl)-N′-phenyl-p-phenylendiamin
N-(1-Methyl-heptyl)-N′-phenyl-p-phenylendiamin
N-Cyclohexyl-N′-phenyl-p-phenylendiamin
4-(p-Toluol-sulfonamido)-diphenylamin
N,N′-Dimethyl-N,N′-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert-butyl-4-dimethylamino-methyl-phenol
2,4′-Diamino-diphenylmethan
4,4′-Diamino-diphenylmethan
N,N,N′,N′-Tetramethyl-4,4′-diamino-diphenylmethan
1,2-Di-[(2-methyl-phenyl)-amino]-ethan
1,2-Di-(phenylamino)-propan
(o-Tolyl)-biguanid
Di-[4-(1′,3′-dimethyl-butyl)-phenyl)amin
tert-octyliertes N-Phenyl-1-naphthylamin
Gemisch aus mono- und dialkylierten tert-Butyl-/tert-Octyldiphenylaminen.

Beispiele für Metallpassivatoren sind:

für Kupfer, z.B.:
Triazol, Benztriazol, und deren Derivate, 2-Mercaptobenzthiazol, 2,5-Di-mercaptothiadiazol, Salicyliden-propylendiamin, Salze von Salicylaminoguanidin.

Beispiele für Rost-Inhibitoren sind:

a) Organische Säuren, ihre Ester, Metallsalze und Anhydride, z.B.: N-Oleoyl-sarcosin, Sorbitan-monooleat, Blei-naphthenat, Dodecenylbernsteinsäure-anhydrid, Alkenylbernsteinsäure-Halbester, 4-Nonylphenoxy-essigsäure.

b) Stickstoffhaltige Verbindungen, z.B.:

I. Primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen, z.B.:
Substituierte Imidazoline und Oxazoline.

c) Phosphorhaltige Verbindungen, z.B.:
Aminsalze von Phosphorsäurepartialestern.

d) Schwefelhaltige Verbindungen, z.B.:
Barium-dinonylnaphthalin-sulfonate, Calciumpetroleum-sulfonate.

Beispiele für Viskositätsindex-Verbesserer sind z.B.
Polymethacrylate, Vinylpyrrolidon/Methacrylat-Copolymere, Polybutene, Olefin-Copolymere, Styrol/Acrylat-Copolymere.

Beispiele für Stockpunkterniedriger sind z.B.:
Polymethacrylat, alkylierte Naphthalinderivate.

Beispiele für Dispergiermittel/Tenside sind z.B.:
Polybutenylbernsteinsäure-imide, Polybutenylphosphonsäurederivate, basische Magnesium-, Calcium-, und Bariumsulfonate und -phenolate.

Beispiele für Verschleisschutz-Additive sind z.B.:
Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldisulfide.

Die erfindungsgemässen Verbindungen dienen insbesondere als Zusätze für Schmiersysteme, insbesondere Motorenöle. Sie weisen in Schmiersystemen Hochdruck-, Antiverschleiss-, Antioxidans- und Korrosionsinhibitor-Wirkungen auf. Ein besonderer Vorteil dieser Verbindungen ist, dass sie im Gegensatz zu Verbindungen mit vergleichbaren Eigenschaften Phosphor- und Zinn-frei sind, wodurch dann auch die Nachverbrennung der Abgase nicht beeinträchtigt wird..

Die erfindungsgemässen Verbindungen der Formel I stellen aber auch, wie bereits erwähnt, sehr gute Hochdruck- und Verschleissschutz-Additive für polare funktionelle Flüssigkeiten dar. Ausserdem wirken sie zusätzlich als Korrosionsschutzmittel.

Die Verbindungen der Formel I können also auch in polaren funktionellen Flüssigkeiten, insbesondere auf der Basis von Wasser, Ethylenglycol, Diethylenglycol, Polyethylenglycol und ihren Abmischungen mit Wasser in homogenem Lösungszustand eingesetzt werden. Ebenso können sie auch in funktionellen Flüssigkeiten auf der Basis von Phosphorsäureestern, bevorzugt Phosphorsäure-arylestern eingesetzt werden. Aber auch kolloidale Systeme sind technisch sehr wertvoll, insbesondere aufgrund ihrer günstigen Dispergier- und Emulgiereigenschaften, z.B. für die Herstellung von Emulsionen und Mikroemulsionen.

Die Verbindungen der Formel I können speziell auch in polaren, komplex aufgebauten funktionellen Flüssigkeiten mit Wasser als vorherrschender Basisflüssigkeit, insbesondere mit einem Wasseranteil von mindestens 50 Gew.%, und gegebenenfalls mit einem Gehalt an niedermolekularen Glycol-Verbindungen, wie z.B. Ethylenglycol, Diethylenglycol oder Thiodiethylenglycol, ferner mit Phosphorsäure sowie einer Base zur Einstellung des pH-Wertes zwischen 7,5 und 12, vorzugsweise bei 8,5, als weitere Komponenten, eingesetzt werden.

Basen zur pH-Regulierung sind z.B. Alkalihydroxide, vorzugsweise Kaliumhydroxid, wasserlösliche organische Basen, vorzugsweise Alkanolamine, insbesondere Ethanolamin. Auch polymere Amine, wie z.B. Polyethylenimin sind für die pH-Regulierung einsetzbar.

Die vorstehende, komplex aufgebaute funktionelle Flüssigkeit kann als weitere Komponenten noch Fettsäuren, vorzugsweise Oelsäure, und gegebenenfalls auch Borsäure enthalten.

Die polaren, komplex aufgebauten funktionellen Flüssigkeiten können noch zusätzliche Additive enthalten, die zugegeben werden, um die Grundeigenschaften von diesen funktionellen Flüssigkeiten noch weiter zu verbessern; dazu gehören: weitere Korrosionsschutzmittel, Schaumbrecher, Enthärter und Biocide.

Beispiele für Korrosionsinhibitoren sind folgende:

a) Organische Säuren, ihre Ester, Ammonium-, Amin-, Alkanolamin- und Metallsalze wie z.B. Benzoesäure, p-tert.-Butyl-benzoesäure, Di-Na-sebacat, Triethanolaminlaurat, Isononansäure, Triethanolaminsalz der p-Toluol-sulfonamidocapronsäure, Triethanolaminsalz der Benzol-sulfonamidocapronsäure, Triethanolaminsalze der 5-Ketocarbonsäureabkömmlinge, wie sie in der EP-A 41 927 beschrieben sind, Natrium-N-Lauroyl-sarcosinat oder Nonylphenoxyessigsäure.

b) Stickstoffhaltige Stoffe wie z.B.:
Fettsäurealkanolamide, Imidazoline wie z.B. 1-Hydroxy-ethyl-2-oleyl-imidazolin, Oxazoline, Triazole wie z.B. Benztriazole oder deren Mannich-Basen-Derivate, Triethanolamine, Fettamine, anorganische Salze wie z.B. Natriumnitrat, und die in der EP-A 46 139 beschriebenen Carboxy-Triazinverbindungen.

c) Phosphorhaltige Stoffe wie z.B.
Aminphosphate, Phosphonsäuren oder anorganische Salze wie z.B. Na-Dihydrogenphosphat oder Zinkphosphat.

d) Schwefehaltige Verbindungen z.B.
Natrium-, Calcium- oder Barium-Petrolsulfonate oder heterocyclische Verbindungen wie z.B. Na-Mercaptobenzthiazol.

Komplexierungsmittel, wie Nitrilotriessigsäure und deren Salze, Schaumbrechende Mittel, wie Silikone wie z.B. Polydimethylsiloxane, Distearylsebacamid, Distearyladipamid und ähnliche von Ethylenoxid-und/oder Propylenoxidkondensationen abgeleitet Produkte, Additionsprodukte von Fettalkoholen, wie Caprylalkohole und deren Kondensationsprodukte mit Ethylenoxid, ferner Biocide wie z.B. Amine, quaternäre Ammoniumverbindungen, Chlorphenole, schwefelhaltige Verbindungen, wie Sulfone, Methylen-bis-thiocyanate und -Carbamate, Isothiazolone, Brompropionamide, Triazine, Phosphoniumverbindungen, Chlor und Chlor abgebende Stoffe und Organometallverbindungen wie Tributyl-zinnoxid können zusätzlich eingesetzt werden.

Für den Einsatz von Verbindungen der Formel I in Hydraulikflüssigkeiten ist die Mitverwendung eines zusätzlichen Verdickers als weitere Komponente vorteilhaft.

Als Verdicker kommen in Frage z.B. Polyalkylenoxide, Polyalkylmethacrylate, Polyamidester, Polyamid-alkoxylate oder Polyethylenimine, die gleichzeitig vollständig oder partiell die Funktion eines pH-Regulators übernehmen können.

Die Einsatzmenge der Verdicker liegt zweckmässig zwischen 2 und 50 Gew.-%. vorzugsweise zwischen 5 und 15 Gew.-%, bezogen auf die Basisflüssigkeit.

Die Einsatzmenge der Verbindungen der Formel I beträft beispielsweise 0,01 bis 10 Gew.-%, vorzugsweise 0,01 bis 5 Gew.-%, z.B. 0,05 bis 3 Gew. % bezogen auf die Gesamtmenge der polaren funktionellen Flüssigkeit.

Werden die Verbindungen der Formel I in komplex aufgebauten funktionellen Flüssigkeiten eingesetzt, so beträgt ihre Einsatzmenge insbesondere 0,5-2 Gew.-%, vorzugsweise jedoch 0,5-1 Gew.-%, bezogen auf die Gesamtmenge der komplex aufgebauten funktionellen Flüssigkeit, wobei die Gesamtmenge der zugesetzten Additive vorzugsweise 0,5-10 Gew.%, insbesondere 2,5-5 Gew.-%, bezogen auf deren Wasseranteil, ausmacht.

Polare funktionelle Flüssigkeiten, insbesondere solche auf der Basis von Wasser, Ethylenglykol, Diethylenglycol, Polyethylenglycol und deren Abmischungen mit Wasser oder auf der Basis von Phosphorsäureestern sowie komplex aufgebaute funktionelle Flüssigkeiten können beispielsweise als Hydraulikflüssigkeiten, Metallbearbeitungsflüssigkeiten, Kühlflüssigkeiten oder als "Drilling-fluids" (Bohrflüssigkeiten), insbesondere als Hydraulikflüssigkeiten, eingesetzt werden.

Auf Grund der vorstehend angegebenen Verwendungsmöglichkeiten der erfindungsgemässen Verbindungen der Formel I betrifft die Erfindung auch Zusammensetzungen, die eine funktionelle Flüssigkeit und mindestens eine Verbindung der Formel I enthalten. In diesen Zusammensetzungen ist die funktionelle Flüssigkeit insbesondere ein Schmierstoff (z.B. ein Motorenöl), Kraftstoff, eine Hydraulik-, Metallbearbeitungs-, Kühl- oder Bohrflüssigkeit. Beispiele für Art und Zusammensetzung solcher Flüssigkeiten sind weiter oben angegeben. Insbesondere sind Zusammensetzungen zu erwähnen, die ein natürliches (z.B. auf Basis von Mineralöl) oder synthetisches Schmieröl oder eine Hydraulikflüssigkeit und mindestens eine Verbindung der Formel I enthalten.

Die Menge an Verbindung der Formel I in den erfindungsgemässen Zusammensetzungen beträgt vorzugsweise 0,01 bis 5 Gew.-%, insbesondere 0,05 bis 3 Gew.-%, bezogen auf die funktionelle Flüssigkeit.

Die nachfolgenden Beispiele erläutern die Erfindung weiter. Prozent und Teile beziehen sich darin, ebenso wie in der übrigen Beschreibung und in den Patentansprüchen, auf das Gewicht, wenn nichts anderes angegeben ist.

Beispiel 1:

Eine mit verdünnter Natronlauge auf pH 13 eingestellte wässrige Lösung von 0,5 Mol Natriumthioglycolat [herzustellen aus 46 g (0,5 Mol) Thioglycolsäure und 40,6 g (~ 0,5 Mol) Natriumhydroxid in 200 ml Wasser] wird unter Rühren bei ca. 50°C mit 108 g (0,5 Mol) tert.-Nonylglycidylthioether so versetzt, dass diese Temperatur erhalten bleibt. Nach beendeter Zugabe wird noch 30 min. auf ca. 60°C erwärmt.

Nach dem Abkühlen wird das teilweise in kristalliner Form ausgeschiedene Reaktionsgemisch mit 70 ml konzentrierter Salzsäure versetzt; es kommt dabei zur Bildung zweier flüssiger Phasen. Die obere organische Phase wird abgetrennt und mit ca. 5 g eines sauren Ionenaustauschers (®Lewatit 1080) zur Vervollständigung der Veresterungsreaktion versetzt.

Nach Abtrennung des Ionenaustauschers erhält man 285,5 g der Verbindung der Formel

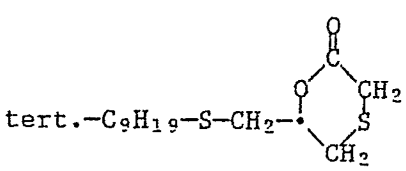

als leicht gelbe Flüssigkeit mit einem $n_D^{20}$ von 1,5222.

Beispiel 2:

In einem 250 ml Dreihalskolben, ausgestattet mit Rührer, Rückflusskühler und Thermometer, werden 53 g (0,5 Mol) Thioglycolsäuremethylester vorgelegt, mit 2 ml Triethylamin versetzt und dann auf ca. 60°C erwärmt. Anschliessend werden 73 g (0,5 Mol) tert.-Butylglycidylthioether zugetropft. Nach beendeter Zugabe wird noch ca. 1 Std. auf 80°C erwärmt, bis schliesslich im DC-Test kein Glycidylthioether mrhr nachweisbar ist.

Durch kurzes Anlegen von Vakuum wird das Triethylamin aus der Reaktionsmischung entfernt. Anschliessend werden ca. 10 g eines sauer eingestellten Ionenaustauschers (®Lewatit 1080) zugesetzt und dann über eine kleine Kolonne das bei der Lactonbildung gebildete Methanol abdestilliert, wobei die Temperatur der Reaktionsmischung allmählich auf ca. 120°C angehoben wird.

Nach dem Abkühlen der Reaktionsmischung wird der Ionenaustauscher abfiltriert und die restlichen flüchtigen Bestandteile mittels Rotationsverdampfer im Vakuum entfernt. Man erhält als Rückstand 125 g der Verbindung der Formel

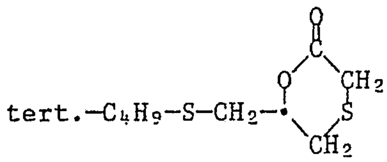

als schwach gelbe Flüssigkeit.

Nach Destillation bei 148-150° C bei einem Druck von 0,25 Torr kristallisiert die Verbindung. Schmelzpunkt: 73° C.

Beispiel 3:

In einem 500 ml Dreihalskolben, ausgerüstet mit Rückflusskühler. Thermometer, Wasserabscheider und Rührer werden 96,2 g der Verbindung der Formel tert.-$C_4H_9$-S-$CH_2CH(OH)$-$CH_2$-S-$CH_2$-COOH, 4 g eines sauren Ionenaustauschers (®Lewatit S 1080) und 250 ml Toluol vorgelegt und das Reaktionsgemisch 140 Minuten unter Rühren auf Rückflusstemperatur erhitzt, wobei das gebildete Reaktionswasser (7,2 ml) über den Wasserabscheider abgetrennt wird. Nach Zugabe weiterer 2 g des Ionenaustauschers werden weitere 1,2 ml Wasser bei Rückflusstemperatur abgeschieden. Dann wird der Ionenaustauscher mit einem Faltenfilter abgetrennt und das Lösungsmittel auf einem Rotationsverdampfer abgetrennt. Nach Destillation des Rückstandes bei 148-150° C und 0,25 Torr erhält man 53,3 g der gleichen Verbindung wie in Beispiel 2 mit einem Schmelzpunkt von 72-73° C.

Beispiel 4:

In einem 1 l-Vierhalskolben, ausgerüstet mit Rührer, Tropftrichter, Thermometer und Rückflusskühler werden 22 g NaOH in 300 ml Wasser gelöst, 46,1 g α-Mercaptoessigsäure eingetropft und anschliessend bei 60-70°C innerhalb 30 Minuten 73,1 g tert.-Butylglycidylthioether zugesetzt (exotherme Reaktion). Das Gemisch wird über Nacht stehen gelassen und dann werden 72,9 g 32%ige HCl zugetropft, die wässrige Phase abgetrennt und einmal mit Diethylether gewaschen. Die vereinigten organischen Phasen werden mit 100 ml Wasser gewaschen, getrocknet und auf Rückstand eingeengt. Man erhält so 98,6 g einer schwach gelben Flüssigkeit mit einem $n_D^{20}$ von 1,5330. Das Produkt entspricht jenem gemäss Beispiel 2 erhaltenen und kann ebenfalls durch Destillation weiter gereinigt werden.

Beispiel 5:

Mit dem Shell-Vierkugel-Apparat (IP 239/73 Extreme pressure and wear lubricant test for oils and greases-four ball-machine) werden folgende Werte bestimmt:

1. W.L. = Weld load (Schweisslast). Das ist die Last, bei der die 4 Kugeln innerhalb von 10 Sekunden zusammenschweissen.
2. W.S.D. = Wear Scar Diameter in mm: Das ist der mittlere Verschleissdurchmesser bei einer Belastung von 400 N während 10 Minuten.

Als Testflüssigkeit für die Wirksamkeit der Additive wird ein Basisöl der Viskosität ISO-VH 100 mit niedrigem Aromatengehalt und 0,035 % S verwendet. Die Konzentration des Additivs im Oel beträgt 1 %.

Die Ergebnisse sind in der folgenden Tabelle 1 zusammengefasst.

Tabelle 1

| Additiv gemäss Beispiel Nr. | W.L. (N) 1 % Additiv | W.S.D. (mm) 1 % Additiv |
|---|---|---|
| keines | 1450 | 0,95 |
| 1 | 2000 | 0,70 |

Beispiel 6: FZG-Test auf EP/AW-Eigenschaften nach DIN 51354. FZG = Forschungsstelle Zahnräder und Getriebe, TU München.

Die Durchführung des Tests nach der genannten Norm kann kurz folgendermassen beschrieben werden: Im Tauschschmierungsverfahren laufen in dem zu prüfenden Schmieröl definierte Zahnräder bei konstanter Drehzahl und festgelegter Anfangs-Oeltemperatur. Die Belastung der Zahnflanken wird stufenweise gesteigert (Kraftstufen 1 bis 12). Ab Kraftstufe 4 wird nach jeder Kraftstufe die Veränderung der Zahnflanken durch Beschreibung, Rauhheitsmessungen oder Kontrastabdrucke festgehalten. Als Mass für die Wirksamkeit des Schmieröls wird diejenige Kraftstufe ermittelt, bei der die Summe der Zahnschäden (Breiten aller Riefen und Fresser der Zahnflanken) des Ritzels mehr als 20 mm beträgt. Diese Kraftstufe wird als Schadenskraftstufe (failure load stage, FLS) bezeichnet. Je höher die Schadenskraftstufe, desto besser die Wirksamkeit des geprüften Schmieröls.

Im vorliegenden Beispiel wird als Grundöl ein Mineralöl VG. 32 verwendet. Zur Prüfung der Wirksamkeit als Additiv werden diesem Grundöl 0,4 % der Verbindung gemäss Beispiel 1 zugesetzt. Die erhaltenen Testergebnisse sind der nachfolgenden Tabelle 2 zu entnehmen.

Tabelle 2

| Additiv aus | FLS |
|---|---|
| —— | 5 |
| Beispiel 1 (0,4 %) | 12 |

## Patentansprüche

1. Verbindungen der Formel

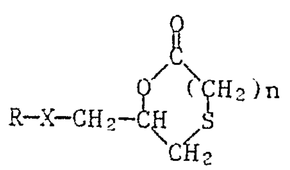

(I)

worin R $C_1$-$C_{24}$-Alkyl, $C_3$-$C_{20}$-Alkenyl, $C_5$-$C_6$-Cycloalkyl, unsubstituiertes oder durch $C_1$-$C_4$-Alkyl substituiertes Phenyl oder Phenyl-$C_1$-$C_4$-alkyl, n 1 oder 2 und X O oder S bedeuten.

2. Verbindungen nach Anspruch 1, worin n 1 bedeuten.

3. Verbindungen nach Anspruch 1 oder 2, worin R $C_1$-$C_{18}$-Alkyl, $C_3$-$C_{18}$-Alkenyl, Phenyl oder Benzyl bedeutet.

4. Verbindungen nach Anspruch 3, worin R $C_4$-$C_{18}$-Alkyl, Phenyl oder Benzyl bedeutet.

5. Verbindungen nach einem der Ansprüche 1 bis 4, worin die Alkylgruppe R mindestens ein tertiäres C-Atom enthält.

6. Verbindungen nach Anspruch 4 oder 5, worin R $C_4$-$C_{18}$-Alkyl bedeutet.

7. Verbindungen nach einem der Ansprüche 1-6, worin X S bedeutet.

8. Zusammensetzungen, enthaltend eine funktionelle Flüssigkeit und mindestens eine der in den Ansprüchen 1 bis 7 definierten Verbindungen der Formel I.

9. Zusammensetzungen nach Anspruch 8, worin die funktionelle Flüssigkeit ein Schmierstoff, Kraftstoff, eine Hydraulikflüssigkeit, Metallbearbeitungsflüssigkeit, Kühlflüssigkeit oder Bohrflüssigkeit ist.

10. Zusammensetzungen nach Anspruch 8, worin die funktionelle Flüssigkeit ein natürliches oder synthetisches Schmieröl oder eine Hydraulikflüssigkeit ist.

11. Zusammensetzungen nach Anspruch 8, die die Verbindungen der Formel I in einer Menge von 0,01

bis 5 Gew.-%, bezogen auf die funktionelle Flüssigkeit, enthalten.

12. Verwendung von in den Ansprüchen 1 bis 7 definierten Verbindungen als Stabilisatoren und Korrosionsschutzmittel in funktionellen Flüssigkeiten.

13. Verwendung nach Anspruch 11 als Verschleissschutz- und Hochdruckadditive in Schmierstoffen und Hydraulikflüssigkeiten.